Europäisches Patentamt

**European Patent Office**

**Office européen des brevets**

(11) Numéro de publication: **0 253 698 B1**

(12) **FASCICULE DE BREVET EUROPEEN**

(45) Date de publication de fascicule du brevet: **20.05.92**

(21) Numéro de dépôt: **87401400.4**

(22) Date de dépôt: **19.06.87**

(51) Int. Cl.⁵: **G01N 33/68**, C07K 7/00, C07K 15/00, A61K 39/00, A61K 37/02, C12Q 1/68

(54) **Procédé d'analyse de séquences peptidiques immunogènes en vue de la production de vaccins.**

(30) Priorité: **19.06.86 FR 8608896**

(43) Date de publication de la demande:
**20.01.88 Bulletin 88/03**

(45) Mention de la délivrance du brevet:
**20.05.92 Bulletin 92/21**

(84) Etats contractants désignés:
**AT BE CH DE ES FR GB GR IT LI LU NL SE**

(56) Documents cités:

**SCIENCE, vol. 229, no. 4717, septembre 1985, pages 932-940, Washington, D.C., US; J.A. BERZOFSKY: "Intrinsic and extrinsic factors in protein antigenic structure"**

**PROCEEDINGS OF THE NATIONAL ACADEMY OF SCIENCE OF THE USA, vol.81, avril 1984, pages 2489-2493; P.M. ALLEN et al.: "Processing of lysozyme by macrophages: Identification of the determinant recognized by two-T-cell hybridomas"**

(73) Titulaire: **INSTITUT PASTEUR**
**25-28, rue du Docteur Roux**
**F-75724 Paris Cédex 15(FR)**

Titulaire: **INSTITUT NATIONAL DE LA SANTE ET DE LA RECHERCHE MEDICALE (INSERM)**
**101, rue de Tolbiac**
**F-75013 Paris(FR)**

(72) Inventeur: **Kourilsky, Philippe**
**26, rue de Montpensier**
**F-75001 Paris(FR)**
Inventeur: **Claverie, Jean-Michel**
**38, rue Cabanis**
**F-75014 Paris(FR)**

(74) Mandataire: **Gutmann, Ernest et al**
**S.C. Ernest Gutmann - Yves Plasseraud 67, boulevard Haussmann**
**F-75008 Paris(FR)**

Rank Xerox (UK) Business Services
(3.08/2.19/2.0)

NUCLEIC ACIDS RESEARCH, vol.14, no. 1, janvier 1986, pages 179-196, IRL Press Ltd, Oxford, GB; J.M. CLAVERIE et al.: "Heuristic informational analysis of sequences"

PROCEEDINGS OF THE NATIONAL ACADEMY OF SCIENCE OF THE USA, vol. 82, octobre 1985, pages 7048-7052, Washington, D.C., US; C. DELISI et al.: "T-cell antigenic sites tend to be amphipathic structures"

PROCEEDNGS OF THE NATIONAL ACADEMY OF SCIENCE OF THE USA, vol. 82, janvier 1985, pages 178-182, Washington, D.C., US; H.M. GEYSEN et al.: "Small peptides induce antibodies with a sequence and structural requirement for binding antigen comparable to antibodies raised against the native protein"

BIOLOGICAL ABSTRACTS, vol. 82, octobre 1986, résumé 92771, Biological abstracts, Inc., Philadelphia, PA, US; P. KOURILSKY et al.: "The peptide self model: a hypothesis on the molecular nature of the immunological self"

## Description

L'invention concerne un procédé d'analyse de ceux des épitopes ou déterminants antigéniques qui, au sein d'une protéine antigénique, peuvent être tenus pour responsables de l'action immunogène de cette protéine vis-à-vis d'un hôte vivant déterminé.

Il est bien connu que l'une des fonctions essentielles du système immunitaire est de reconnaître ce qui lui est étranger, pour l'éliminer, cependant sans s'attaquer aux protéines de l'hôte. Celles-ci ou l'ensemble des peptides qui résultent de la fragmentation au hasard des protéines de l'hôte seront dans ce qui suit désignés par l'expression le "soi somatique".

Une forme de défense courante par laquelle le système immunitaire se manifeste vis-à-vis d'une protéine étrangère consiste en la capacité qu'il a de produire des anticorps reconnaissant spécifiquement certains sites antigéniques ou épitopes de cette protéine. Ces épitopes sont souvent portés par des séquences peptidiques particulières relativement courtes, lesquelles ne constituent le plus souvent que des parties très petites de la séquence en acides aminés de la protéine antigénique entière.

Peu de moyens sont à l'heure actuelle disponibles pour déterminer la position et la nature de l'épitope ou des épitopes porteurs de l'immunogénicité de la protéine, même lorsque la séquence entière de cette protéine est connue ou peut être déduite de la séquence nucléotidique de l'ADN codant pour cette protéine, lorsque cet ADN particulier a été identifié. On mentionnera pour mémoire les mesures ou appréciations classiques qui sont faites des degrès d'hydrophilie de certaines régions de la séquence peptidique totale de la protéine concernée ou les tentatives que l'on fait quelquefois de les situer sur des boucles dont on pense qu'elles pourraient être exposées à la surface de l'antigène. Les résultats que fournissent ces méthodes sont souvent décevants. Tant que l'on ne disposera pas de procédés d'analyse plus rapides et/ou efficaces des épitopes caractéristiques de ces protéines, le développement de vaccins synthétiques risque d'être freiné.

Dans la publication de Nucleic Acids Research (ref. 5) CLAVERIE et al décrivent le fait que des séquences d'acides aminés ou de nucléotides sont interprétées comme étant des succesions de k-tuplets de fréquence variable selon les populations de gènes ou de protéines.

A partir de cette hypothèse les auteurs réalisent des études sur différentes séquences, en comparant des X-tuplets formant ces séquences avec les enchaînements de même longueur disponibles dans des banques de données. Ceci permet de connaître la fréquence de différents X-tuplets donnés et d'envisager que les fragments à fonction particulière de protéines sont constitués par des séquences statistiquement rares.

Dans l'article de Science (vol. 229, p.932-940) BERZOFSKY s'intéresse à la prédiction des sites antigéniques reconnus par les anticorps et les cellules T et étudie pour cela l'influence des facteurs intrinsèques à l'antigène et des facteurs dépendants de l'hôte qui déterminent les épitopes spécifiques de la réponse immunitaire.

D'après BERZOFSKY les facteurs intervenant forment un ensemble et il conclut que en théorie toute la surface accessible d'une protéine peut être antigénique, que des facteurs intrinsèques et des facteurs extrinsèques interviennent néanmoins dans l'antigénicité.

On sait que de nombreux peptides, dont la séquence d'acides aminés est contenue dans celle de la protéine contre laquelle une protection est recherchée, ont été synthétisés et testés dans des réactions immunologiques mettant en jeu des anticorps préalablement formés contre la protéine. S'il a souvent été constaté que ces anticorps reconnaissaient ces polypeptides synthétiques, la capacité que possèdent ces derniers d'induire in vivo des anticorps protecteurs contre la protéine naturelle n'a été constatée que dans un nombre réduit de cas, et cela même lorsque ces polypeptides avaient au préalable été rendus efficacement immunogènes, par exemple par fixation sur un support macromoléculaire approprié.

L'invention a pour but de remédier aux difficultés rencontrées jusqu'à ce jour, à l'occasion de l'analyse des séquences peptidiques éventuellement responsables de l'immunogénicité d'une protéine antigénique à l'égard d'un hôte déterminé, dès lors que sa séquence en aminoacides a été réalisée ou peut-être déduite de la séquence nucléotidique de l'ADN correspondant, dès lors qu'elle aura été déterminée au préalable.

L'invention découle d'une approche différente des problèmes de l'immunité. D'une façon générale, il est admis que les épitopes des protéines antigéniques, susceptibles d'induire des anticorps in vivo correspondent à des portions de molécules normalement exposées à la surface de ces protéines ou de cellules, en particulier lorsque l'antigène est constitué par un micro-organisme ou tout autre type cellulaire étranger. Ces théories reposent sur l'hypothèse qui avait été faite que, pour être recoonus par le système immunitaire, les épitopes devaient être exposés à la surface de l'antigène et au contact des fluides biologiques circulants.

L'approche différente que l'invention met en oeuvre est en rapport avec une interprétation nouvelle que les inventeurs font des progrès importants qui ont récemment été réalisés au niveau de la compréhension de la présentation des antigènes par des molécules de classe I et de classe II codées par le complexe majeur d'histocompatibilité. Tout d'abord, il a été constaté que les antigènes de classe II exposés à la surface des macrophages sont capables de présenter d'une certaine manière des peptides à des cellules auxiliaires T (réf. 8). Ainsi le lysozyme de blanc d'oeuf est-il résolu en peptides, dont certains peuvent être présentés par des antigènes de classe II (réf. 2). Il a également été montré que la nucléoprotéine de la grippe est probablement présentée sous forme de petits peptides aux lymphocytes T cytotoxiques, en association avec des molécules de classe I (réf. 12 et 13). Dans les deux cas, des peptides correspondants ont été synthétisés in vitro et mis en contact avec des cellules qui présentaient des antigènes de classe I ou de classe II à leur surface. Il devait alors être constaté que ces peptides acquéraient la capacité d'être présentés d'une façon spécifique les rendant reconnaissables par le système immunitaire.

L'interprétation que les auteurs ont donné de cette expérience est qu'en fait le lysozyme et la nucléoprotéine de la grippe avaient subi une dégradation intracellulaire, laquelle s'était manifestée par la production des peptides. Ceux-ci avaient alors été transférés à la surface des cellules en cause pour être présentés sous la forme appropriée au sytème immunitaire par les antigènes de classe II et de classe I. De là découle l'hypothèse supplémentaire formulée par les inventeurs que l'ensemble des protéines cellulaires, étrangères ou non, peut subir cette dégradation en peptides, suivie de leur présentation à la surface. Il en résulte que le système immunitaire doit être capable de reconnaître des séquences peptidiques étrangères à l'ensemble des séquences peptidiques appartenant au soi somatique de l'hôte.

L'invention repose donc sur le principe d'une recherche dans la protéine antigénique à l'égard de laquelle une vaccination peut être recherchée de ceux des peptides qui, par exemple au terme d'un processus comprenant l'internalisation du complexe protéine antigèneanticorps dans des lymphocytes B, la fragmentation au hasard de la protéine en petits peptides et le transfert de ces derniers à la surface de ces cellules, pourraient être reconnus par les cellules possédant des récepteurs T comme étrangers vis-à-vis de l'ensemble des peptides du "soi somatique".

En d'autres termes, l'invention peut être considérée comme découlant de l'hypothèse que l'antigénicité de la protéine antigénique devait être due à des peptides que le système immunitaire n'avait pas jusque là appris à reconnaître comme appartenant au moi somatique de l'hôte.

Le procédé sedon l'invention pour l'analyse au sein d'une protéine étrangère à l'organisme d'un hôte appartenant à une espèce déterminée de la séquence peptidique portant l'épitope responsable de l'immunogénicité qui pourrait être mise en oeuvre pour la fabrication ultérieure de peptides ou polypeptides immunogènes ayant des propriétés vaccinantes contre la protéine étrangère ou contre l'antigène qui la comporte est caractérisé par la combinaison d'étapes que constituent :

- la détermination et l'identification de la famille des oligopeptides ayant une longueur déterminée, en l'occurrence, supérieure à 3 acides aminés, qui sont contenus dans la séquence en acides aminés constitutifs de cette protéine étrangère,
- la comparaison de chacun des oligopeptides de cette famille avec chacun des oligopeptides de mêmes longueurs de l'ensemble des familles d'oligopeptides contenus dans les protéines dont les séquences en acides aminés sont connues et qui sont disponibles dans un catalogue préétabli de ces protéines, toutes ces protéines étant caractéristiques du "soi somatique" de l'espèce correspondante,
- l'identification de ceux des oligopeptides (oligopeptides non coïncidents) de la famille de la protéine étrangère pour lesquels n'existent que peu de coïncidences et, de préférence, aucune coïncidence avec les oligopeptides disponibles dans le catalogue,
- la recherche, au sein de la protéine étangère de séquences formées d'oligopeptides non coïncidents qui se chevauchent mutuellement, et
- l'analyse parmi ces séquences formées d'oligopeptides successifs non coïncidents et se chevauchant mutuellement d'au moins une séquence d'acides aminés portant l'épitope requis pour la fabrication du polypeptide vaccinant.

Il doit être entendu que dans ce qui précède "oligopeptide ayant une longeur déterminée" fait référence à un peptide ayant un nombre déterminée d'acides aminés, naturellement quelles que soient leurs natures respectives. Dans les exemples qui suivent, les "oligopeptides" possèdent respectivement 4 aminoacides.

Le "catalogue" dont il a également été question plus haut, correspond à toutes banques de données dans lesquelles sont répertoriées les séquences en acides aminés de protéines en nombre de plus en plus important. L'expression s'applique également aux banques de données dans lesquelles sont rassemblées toutes les informations relatives aux séquences nucléotidiques des fragments d'ADNs séquencés, parmi lesquels de nombreux ADNs codant des protéines dont les séquences en acides aminés peuvent alors être déduites au moyen du code génétique.

4

On comprendra que toute protéine formée d'un nombre x d'aminoacides contient x - 3 tétrapeptides, selon la définition qui a été donnée de l'invention. Si l'on part de l'extrémité N terminale de la protéine, le premier des tétrapeptides du genre en question sera formé à partir des 4 premiers aminoacides à compter de l'extrémité N terminale, le second oligopeptide ou tétrapeptide s'étendra entre le deuxième et le cinquième acide aminé de la séquence de la protéine totale, etc. et le dernier du (x - 3)$^{\text{ème}}$ à l'aminoacide C-terminal. Il en ira évidemment de même pour les différentes familles d'oligopeptides, plus particulièrement de tétrapeptides qui pourront être identifiés dans chacune des protéines appartenant à l'espèce déterminée étudiée, dont les séquences en acides aminés sont connues et répertoriées dans la banque de données utilisée.

Le choix des "tétrapeptides" dans les exemples décrits plus loin s'explique par la grande quantité de tétrapeptides qui peuvent être déjà déduits des séquences en acids aminés de l'ensemble des protéines dont les structures sont déjà connues et qui proviennent toutes d'une même espèce. Il existe en effet $20^4$ = 160.000 différentes combinaisons d'aminoacides dans un tétrapeptide.

Partant de ces données, il a été remarquable de constater en rapport avec certains polypeptides de synthèse dont avait déjà été montrée la capacité d'induire in vivo des anticorps reconnaissant la protéine entière correspondante, que ces polypeptides se sont finalement avérés contenir des oligopeptides, plus particulièrement des tétrapeptides, qui n'avaient pas leur équivalent au sein des ensembles des familles d'oligopeptides contenues dans les protéines, dont les séquences en acides aminés sont disponibles dans la banque de données, qui a été utilisée en rapport avec les exemples décrits plus loin. Il a même été constaté que cette absence de communauté entre oligopeptides de la protéine antigénique envisagée et oligopeptides, notamment tétrapeptides, des protéines répertoriées dans la banque de données s'étendait souvent à des oligopeptides successifs se chevauchant mutuellement, c'est-à-dire décalées dans la séquence de la protéine chaque fois d'un aminoacide, l'un vis-à-vis de l'autre. Cette constatation donne donc un fondement raisonnable à l'hypothèse que les séquences peptidiques "non coïncidentes" correspondantes pouvaient correspondre à ceux des oligopeptides, qui pouvaient résulter de la fragmentation au hasard de la protéine antigénique, à l'occasion du processus rappelé plus haut de détection de la protéine antigénique in vivo en vue de sa destruction.

Le choix de tétrapeptides pour la réalisation des essais décrits plus loin a essentiellement été dicté par l'étendue des informations disponibles à l'heure actuelle quant aux séquences en acides aminés de protéines appartenant à une espèce donnée. On mentionnera par exemple que le total des acides aminés contenus dans les séquences des protéines de souris répertoriées dans la banque utilisée était de 26.000 pour les protéines "somatiques" de la souris et de 16.000 pour les protéines "somatiques" du lapin.

Il va sans dire que lorsque les données relatives aux séquences en aminoacides des protéines disponibles dans les banques se seront suffisamment accrues, les oligopeptides utilisables pour la mise en oeuvre du procédé selon l'invention pourront être constitués par des pentapeptides, des hexapeptides, voire même des oligopeptides de plus grandes longueurs. A l'heure actuelle, les séquences disponibles dans les banques de données sont encore insuffisantes pour permettre une analyse statistiquement significative de la distribution ou au contraire de l'absence de distribution de certains pentapeptides ou hexapeptides entre la protéine étudiée, d'une part, et l'ensemble des familles d'oligopeptides contenues dans les protéines "somatiques" de l'hôte, d'autre part. A l'inverse l'utilisation de séquences tripeptidiques ne permettait plus la mise en évidence significative de tripeptides "non coïncidents" dans la protéine antigénique étudiée et non présente parmi les protéines "somatiques" de l'hôte.

Les familles d'oligopeptides du "soi somatique", disponibles dans les banques de données et vis-à-vis desquelles la comparaison sus-indiquée est effectuée, peuvent provenir de la totalité des protéines de l'hôte disponibles dans ces banques de données, ou le cas échéant être dépourvues de celles des protéines intervenant directement au niveau du système immunitaire : séquences connus des anticorps, des récepteurs de cellules T, molécules de classe I et de classe II, etc.. Il a en effet été constaté que ce dernier type de protéines contenaient de nombreux tétrapeptides non coïncidents avec ceux des autres protéines au sein de la même espèce. Cette observation donne donc une certaine crédibilité à la notion qu'il existe chez tout individu un "soi somatique" et un "soi immunitaire" qui ne coïncident pas.

Le principe du procédé selon l'invention sera maintenant illustré par la description de certains exemples. Il sera fait référence aux dessins dans lesquels :
- La fig. 1 fournit un graphique représentatif de la fréquence de distribution commune de mêmes tétrapeptides d'une nucléoprotéine de la grippe, d'une part, et dans l'ensemble des protéines séquencées de souris qui, au moment où l'essai a été réalisé, étaient disponibles dans la banque de données NBRF-PIR.

- La fig. 2 fournit des courbes fournissant les mêmes informations comparatives, cette fois-ci entre les tétrapeptides présents dans la structure d'un lysozyme, d'une part, et dans l'ensemble des protéines de la banquue de données sus-indiquée, d'autre part.
- La fig. 3 fournit les mêmes donnée comparatives entre la séquence d'un fragment N- terminal de la protéine VP1 du poliovirus de type 1, d'une part, et les données disponibles relatives aux protéines du lapin dans la même banque de données, d'autre part.

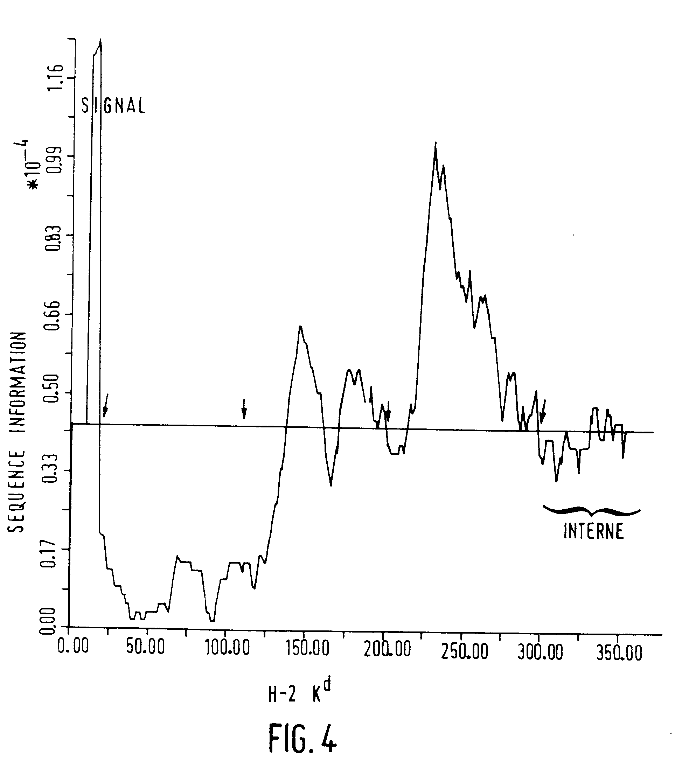

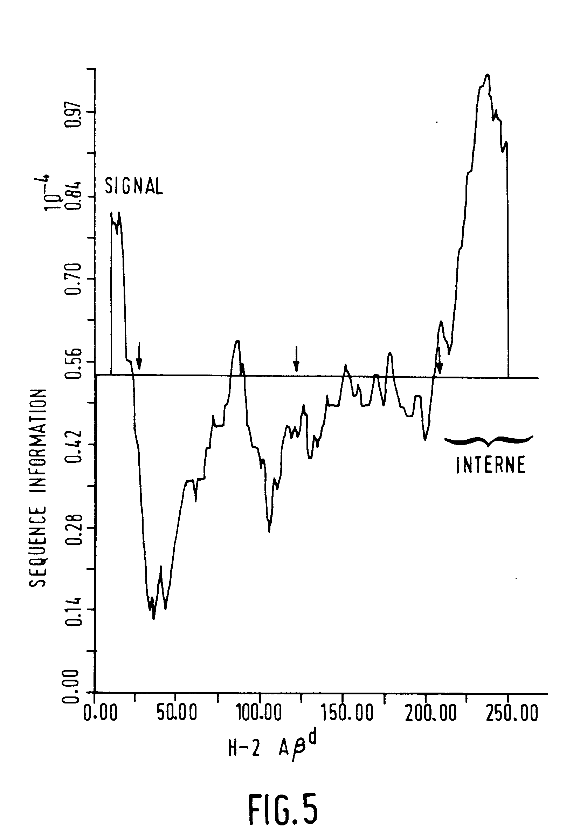

Enfin les fig. 4 et 5 fournissent des données comparatives entre des molécules de classes I et II de la souris et la banque de données relatives aux familles d'oligopeptides qui peuvent être déduites des séquences des protéines de souris de la même banque de données.

EXAMPLE I : Etude d'un peptide immunogène de la nucléoprotéine A/MT/60/68 de la grippe par rapport à une base de données relative au "soi somatique" de la souris.

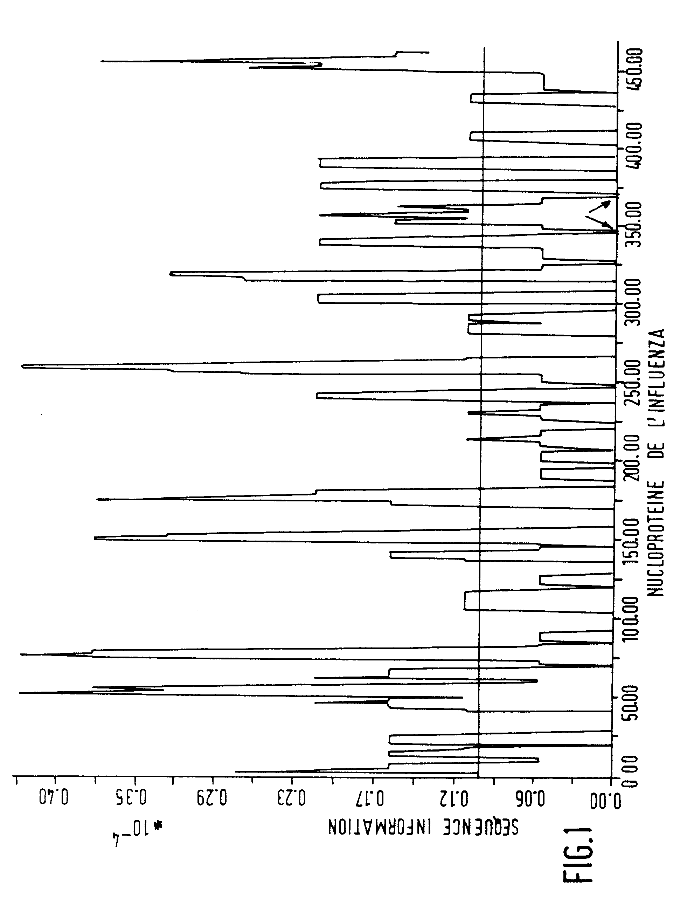

La fig. 1 permet l'évaluation de la fréquence d'existence dans la base de données de chacun des tétrapeptides qui se trouvent être inclus dans la nucléoprotéine de la grippe, et ce à partir de la première séquence tétrapeptidique à compter de l'acide aminé N-terminal de la nucléoprotéine (positon) 0.00 sur l'axe des abscisses). La fréquence d'apparition des fréquences correspondantes est appréciable vis-à-vis de l'axe des ordonnées. Les variations relatives qui apparaissent à la fig. 1 correspondent à la fréquence de détection dans la base de données de chacun des tétrapeptides chevauchants, décalés les uns par rapport aux autres d'un aminoacide de la séquence de nucléoprotéines. Ainsi évalue-t'on les fréquences d'apparition des tétrapeptides LVWM, VWMA, WMAC ...... à partir d'une séquence LVWMAC ......, elle-même contenue dans la nucléoprotéine de la grippe. Les nombres apparaissant sous l'axe des abscisses correspondent aux numérotations des aminoacides N-terminaux successifs des tétrapeptides successifs vis-à-vis de l'acide aminé N-terminal de la nucléoprotéine.

La fig. 1 fait apparaître 25 régions de la séquence dela nucléoprotéine dans lesquelles on constate la présence d'au moins 7 tétrapeptides dont les premiers acides aminés respectifs se suivent les uns les autres, tous absents de l'ensemble des tétrapeptides déductibles de la base de données. Il est à cet égard intéressant de remarquer que deux régions de la séquence (repérées dans la fig. 1 par des flèches et s'étendant entre les résidus 335 et 349, d'une part, et entre 365 et 379, d'autre part) coïncident avec deux régions de la séquence dont avait auparavant été démontrée la capacité de jouer un rôle au niveau de la reconnaissance par les lymphocytes T cytotoxiques de la souris.

Ces parties de la séquence d'aminoacides apparaît également dans le tableau I ci-après (alinéa a)). Comme on pourra le constater, le tableau I illustre :

1) une partie de la séquence en acides aminés de la nucléoprotéine (s'étendant entre les résidus 328 et 385) en utilisant le code à une lettre pour la désignation des acides aminés : en lettres majuscules ;

2) En traits interrompus les peptides de synthèse dont question plus haut, étant entendu que chacun des tirets des traits interrompus correspond aux mêmes aminoacides que celui qui se trouve situé à son aplomb dans la première ligne;

3) les séquences non coïncidentes résultant de la comparaison effectuée par le procédé selon l'invention entre ces peptides de synthèse et les séquences peptidiques déductibles de la banque de données, en utilisant également le même code à une lettre pour la désignation des aminoacides : en lettres minuscules.

Il est intéressant de constater que les séquences en lettres minuscules correspondent à des tétrapeptides se chevauchant successivement dont aucun équivalent n'a pour l'instant encore été retrouvé dans la banque de données concernant les protéines de de souris.

En d'autres terms, les peptides à l'égard desquels l'intervention des lymphocytes T cytotoxiques avait déjà été constatée et les séquences non coïncidentes avec celles du "soi somatique" témoignent au contraire d'une coïncidence remarquable entre elles.

EXAMPLE II : le lysozyme de blanc d'oeuf

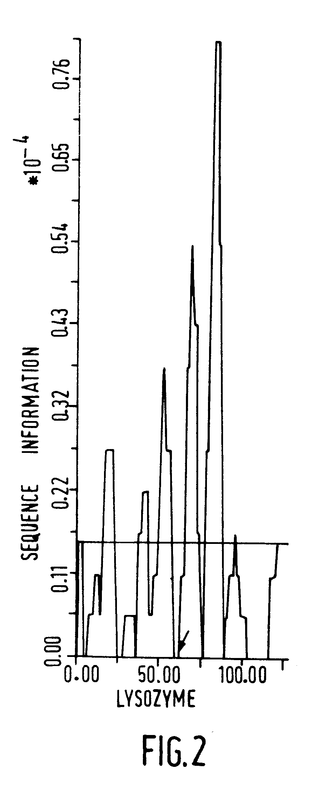

Comme on l'a déjà mentionné plus haut, l'immunisation de souris avec du lysozyme de blanc d'oeuf de poule comprend un processus d'absorption de la protéine par les macrophages et la présentation d'un peptide (résidus 45 à 61) par les antigènes de classe II (réf. 2). Le résultat des opérations de comparaison entre la séquence en acides aminés et la banque du "soi somatique" de la souris résulte de la figure 2. Celle-ci fait de nouveau apparaître une séquence de 9 tétrapeptides consécutifs non coïncidents avec les séqueences de référence de la souris. Le petide 45-61 s'est de nouveau révélé présenter un degré de coïncidence remarquable avec une séquence du lysozyme ne coïncidant pas elle-même avec l'une quelconque des séquences du "soi somatique" de la banque de données.

EXEMPLE III : protéine M de Streptococcus pyrogenes

Un peptide obtenu par la méthode au bromure de cyanogène à partir de la protéine M24 est connu comme étant capable de provoquer une réponse humorale et à déclencher une réaction des lymphocytes T cytotoxiques chez le lapin (réf. 6). Le plus court des peptides synthétiques connus comme provoquant une réponse de la part des lymphocytes T cytotoxiques est de nouveau présenté par un trait interrompu dans le tableau I, chacun des tirets du trait interrompu identifiant l'aminoacide correspondant par référence à la séquence plus longue représentée en utilisant un code à une lettre (lettres majuscules). Comme dans les cas précédents, ce peptide, qui comporte 12 aminoacides s'est révélé inclure une séquence de 9 résidus aminoacides formés de 6 tétrapeptides consécutifs se chevauchant mutuellement, tous ces peptides s'étant révélés absents des informations contenues dans la banque de données quant aux séquences en aminoacides des protéines du "soi somatique" du lapin.

EXEMPLE IV : peptides synthétiques immunogènes issus de l'antigène HBsAg du virus de l'hépatite B

Les séquences de deux peptides immunogènes du virus de l'hépatite B sont indiquées dans le tableau I joint, selon la méthodologie déjà décrite. Il est connu que ces peptides sont capables de provoquer une réponse humorale chez le lapin, le plus long des deux étant le lus immunogénique (réf. 3). Là encore les deux peptides coïncident avec une séquence faite de tétrapeptides se chevauchant mutuellement, dont à l'heure actuelle des équivalents n'ont pas été retrouvés dans la banque du "soi somatique" du lapin. On remarquera d'ailleurs que le peptide le actif est également celui qui comprend la séquence la plus importante, en coïncidence avec une séquence absente du "soi somatique".

EXEMPLE V : peptides synthétiques correspondant à des séquences de la protéine VP1 du poliovirus

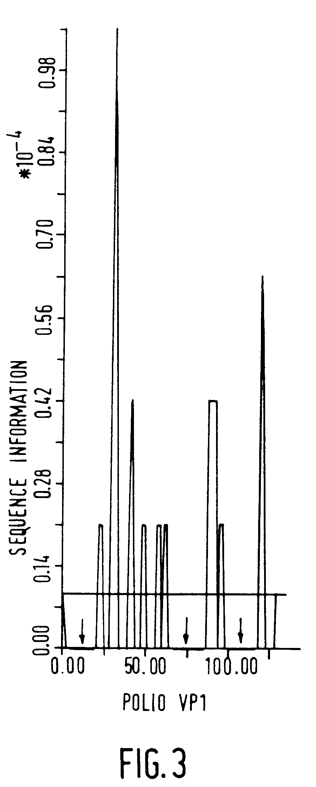

Plusieurs peptides du fragment N-terminal de la protéine VP1 du poliovirus de type I ont été reconnus comme possédant la capacité de provoquer une réponse anti-VP1 chez le lapin (réf. 7). La fig. 3 fait de nouveau apparaître une coïncidence remarquable entre les positions de ces peptides synthétiques avec les séquences les plus longues absentes à l'heure actuelle de la banque du "soi somatique" du lapin.

EXEMPLE VI : molécules de classe I et de classe II de la souris

Les séquences d'aminoacides de lymphocytes de classe I H-2K$^d$ et de la chaîne A$^d$ de l'antigène de classe II (réf. 9 et 10) ont été analysées par la même procédure. Les fig. 4 et 5 font apparaître des propriétés intéressantes. Ces molécules comportent des régions en commun avec les séquences de protéines communes du "soi somatique". Tel est le cas pour une séquence signal impliquée dans le transport de ces molécules à la surface de la membrane à travers les régions cytoplasmiques, ainsi que pour des protéines des trosièmes domaines extracellulaires de H-2K$^d$ (fig. 4). Au contraire, les autres domaines extracellulaires, en particulier les régions NH2-terminales de ces deux molécules présentent un caractère remarquablement "étranger" par rapport à l'ensemble des oligopeptides du "soi somatique" de la souris, tels qu'ils résultent de la banque de données.

Les conclusions générales qui peuvent être tirées des exemples précédents sont les suivantes.

Il est remarquable que dans tous les cas on ait observé un non-chevauchement ou une "non-coïncidence" significatif entre les séquences du peptide dont on avait antérieurement reconnu le caractère immunogénique et les séquences d'aminoacides telles qu'elles résultent des figures, qui correspondent à des séquences formées de tétrapeptides chevauchants que l'on ne retrouvait pas dans les banques de données concernant les protéines du "soi somatique" de l'hôte correspondant.

Les domaines extracellulaires des antigènes du complexe majeur d'histocompatibilité de souris de classe I et de classe II qui interviennent sans doute dans le mécanisme de reconnaissance cellule-cellule contiennent des séquences qui ne sont pas non plus déductibles des familles de tétrapeptides du "soi somatique" de la souris.

Bien que les résultats n'aient été obtenus que par comparaison avec les séquences d'un nombre limité de protéines disponibles dans les banques de données (100 à 200 protéines pour la souris et l'homme), il est légitime de penser que les épitopes caractéristiques des protéines antigéniques à l'égard d'un hôte donné resteront constitués par des séquences d'aminoacides dont on ne retrouvera pas d'équivalents dans l'ensemble des protéines formant le "soi somatique" de l'hôte. Le développpement des informations relatives aux séquences des protéines du "moi somatique" aura certainement pour effet de réduire le nombre des séquences contenues à l'intérieur des futures protéines antigéniques étudiées dont on ne retrouvera pas l'équivalent dans les protéines du "moi somatique" de l'hôte correspondant. Les hypothèses formulées ci-dessus paraissent néanmoins devoir conserver leur valeur, ne serait-ce que par le caractère "étranger" évident qui résulte de l'introduction dans l'organisme de l'hôte d'une séquence peptidique qui lui est manifestement étrangère.

Avantageusement, le procédé selon l'invention pour détecter les zones d'une protéine susceptibles de porter un épitope caractéristique est complété par des étapes supplémentaires consistant dans la synthèse des peptides correspondant aux séquences non communes présumées porter l'épitope susdit, leur utilisation pour l'immunisation d'un hôte vivant approprié, étranger à l'espèce étudiée, avec ces peptides synthétiques et la sélection parmi ceux de ces peptides synthétiques qui auront montré leur capacité à induire in vivo la production d'anticorps capables non seulement de les neutraliser eux-mêmes mais encore de neutraliser les protéines entières correspondantes. Ces polypeptides pourront donc être considérés comme contenant un épitope caractéristique de cette protéine.

Naturellement le procédé selon l'invention peut également être couplé avec les méthodes de reconnaissance déjà appliquées pour identifier des épitopes vraisemblables (par exemple appréciation des degrés d'hydrophilie des régions concernées) qui permettront un premier tri dans le cas où la comparaison sus-indiquée ferait apparaître un nombre relativement important "de séquences non coïncidentes" entre la protéine antigénique étudiée et l'ensemble des protéines du "soi somatique" disponibles dans les banques de données.

Il résulte de l'examen même des figures que le procédé selon l'invention permet, à l'examen de la séquence en aminoacides d'une protéine antigénique de grande longueur, de rapidement situer celles des régions qui pourraient éventuellement contenir un épitope caractéristique de la protéine, à tout le moins d'éliminer avec un grand niveau de certitude les séquences en général beaucoup plus longues qui ne devraient pas le porter.

L'invention sera donc d'un usage particulièrement avantageux pour l'étude des régions susceptibles de porter des épitopes de toute protéine antigénique nouvellement séquencée.

A l'inverse, l'invention permet de déterminer, en quelque sorte par anticipation, quelles seront les régions susceptibles de porter l'épitope dans toute protéine dont la séquence en aminoacides n'a pas encore été faite, dans la mesure où l'on disposerait néanmoins de la région du gène codant pour cette protéine et/ou cette région de gène aurait elle-même fait l'objet d'un séquençage chimique, et ce par le truchement du code génétique.

D'une façon générale, il y a lieu d'observer que l'on pourra éventuellement retenir comme région non coïncidente préférée, celle d'entre elles qui se révèlerait formée d'oligopeptides se chevauchant et comportant en tout de 6 à 30 acides aminés. De tels peptides sont aisément accessibles à la synthèse chimique ou par des procédés du génie génétique. Dès lors qu'ils se révèleraient porter un épitope caractéristique de la protéine étudiée, ils pourraient être directement mis en oeuvre dans la fabrication de principes actifs immunogènes, voire vaccinants contre la protéine entière correspondante, le cas échéant après amplification de leur caractère immunogène dans des conditions bien connues de l'homme du métier, par exemple par couplage avec une molécule porteuse, telle qu'une anatoxine tétanique ou diphtérique ou une sérum albumine, ou encore par association ou couplage avec une molécule possédant des propriétés d'adjuvant immunologique.

Une généralisation de ce qui précède provient de ce que les molécules du système immunitaire, particulièrement les anticorps et les récepteurs des cellules T, peuvent être dégradées en peptides présentés à la surface des cellules du sytème immunitaire, où ils joueraient un rôle régulateur par le biais de leur intervention dans les interactions entre cellules. Ainsi, selon que les régions variables des immunoglobulines ou des récepteurs des cellules T produisent des peptides qui ressemblent ou non au "soi somatique", des signaux régulateurs de nature très différente seront obtenus. L'emploi de peptides ou de polypeptides ainsi définis et synthétisés par voie chimique (ou par des techniques du génie génétique) est donc de nature à moduler l'équilibre du système, notamment par le biais des régulations dites idiotypiques. En d'autres termes, l'invention s'étend aussi aux peptides ou polypeptides tirés des molécules du système immunitaire qui pourront servir, soit de vaccins, soit d'adjuvants à des vaccins, dans des maladies liées soit à l'infection par des agents externes (virus, bactéries, parasites), soit à des désordres internes (maladies auto-immunes, cancers).

TABLEAU I : Corrélation des positions des peptides synthétiques immunogènes ou des séquences caractéristiques rares ou absentes dans les familles de protéines somatiques de lasouris et du lapin, respectivement telles qu'elles sont disponibles dans la banque de données.

a) Nucléoprotéine de la grippe

```
      330       340       350       360       370       380
     LVWMACNSA AFEDL RVLSFIRGTKVSPRGKLSTR GVQIA SNENMDA MESSTLELRSR
                      ---------------             ---------------
                      rvlsfirgt                   asnenmdam
```

b) Lysozyme du blanc d'oeuf de poule

```
        50        60
     RNTDGSTDYGILQINSRWWCNDGRT
                ---------------
                lqinsrwwcndgr
```

c) Protéine M de Streptococcus pyrogenes

```
     NFSTADDSAKIKTLEAEKAALAARKADLEKALEGAM
                           -------------
                           laarkadle
```

d) Antigène HBS

```
     STGPSKTCMTTAQGTSMYPSC
     ---------------------
        -----------------
     stgpsktcmtt
```

e) Protéine VP1 du poliovirus

```
    1  GLGQMLESMIDNTVRETVGAATSRDALPNTEASGPTHSKEIPALTAVETGATNPLVPSDT
              -------
           gqmlesmidntvretvga

   61  VQTRHVVQHRSRSESSIESFFARGACVTIMTVDNPASTTNKDKLFAVWKITYKDTVQLRR
              ------                        -------
           ----------
           vvqhrsrsessiesffarga               ttnkdklfavwkitykdtvq
```

EP 0 253 698 B1

**Revendications**

1. Procédé d'analyse de séquences peptidiques portant un épitope caractéristique d'une protéine étrangère à l'organisme d'un hôte appartenant à une espèce déterminée, pour la fabrication ultérieure de peptides ou de polypeptides immunogènes contenant ces séquences peptidiques et ayant des propriétés vaccinantes contre cette protéine étrangère ou contre l'antigène qui la comporte, caractérisé par la combinaison d'étapes que constituent :
   - la détermination et l'identification de la famille des oligopeptides ayant une longueur déterminée, qui sont contenus dans la séquence en acides aminés constitutifs de cette protéine étrangère,
   - la comparaison de chacun des oligopeptides de cette famille avec chacun des oligopeptides de mêmes longueurs de l'ensemble des familles d'oligopeptides contenus dans les protéines dont les séquences en acides aminés sont connues et qui sont disponibles dans un catalogue préétabli de ces protéines, toutes ces protéines étant caractéristiques du "soi somatique" de l'espèce correspondante,
   - l'identification de ceux des oligopeptides (oligopeptides non coïncidents) de la famille de la protéine étrangère pour lesquels n'existent que peu de coïncidences et, de préférence, aucune coïncidence avec les oligopeptides disponibles dans le catalogue,
   - la recherche, au sein de la protéine étrangère de séquences formées d'oligopeptides non coïncidents qui se chevauchent mutuellement, et
   - l'analyse parmi ces séquences d'oligopeptides non coïncidents et se chevauchant d'au moins une séquence d'acides aminés portant l'épitope requis pour la fabrication du polypeptide vaccinant. portant l'épitope requis pour la fabrication du polypeptide vaccinant.

2. Procédé selon la revendication 1, caractérisé en ce que les susdits oligopeptides sont des tétrapeptides.

3. Procédé selon la revendication 1 ou la revendication 2, caractérisé en ce que l'étape de recherche de séquences formées d'oligopeptides se chevauchant comprend la détermination de celles d'entre elles qui comprennent de 6 à 50 (notamment de 6 à 30) acides aminés.

4. Procédé selon l'une quelconque des revendications 1 à 3, caractérisé en ce que l'étape de détermination et d'identification de la famille des oligopeptides de ladite protéine étrangère comprend le séquençage chimique de cette protéine étrangère, l'identification de ladite famille d'oligopeptides faisant alors immédiatement suite à ce séquençage chimique.

5. Procédé selon l'une quelconque des revendications 1 à 3, caractérisé en ce que l'étape de détermination de la famille des oligopeptides de ladite protéine étrangère comprend le séquençage chimique de la séquence polynucléotique préalablement reconnue comme codant pour ladite protéine étrangère, l'identification de ladite famille d'oligopeptides faisant alors immédiatement suite, par l'intermédiaire du code génétique, au séquençage chimique de la séquence polynucléotidique.

6. Procédé selon l'une quelconque des revendications 1 à 5, caractérisé en ce que l'étape d'analyse parmi les séquences formées d'oligopeptides successifs se chevauchant mutuellement et non coïncidents, d'une ou plusieurs de ces séquences d'acides aminés présumées porter l'épitope susdit, comprend la synthèse chimique d'une ou plusieurs de ces séquences formées d'oligopeptides non chevauchants, l'immunisation d'un hôte vivant approprié, étranger à l'espèce étudiée, avec ces séquences formées d'oligopeptides et la sélection de celles des séquences qui contiennent l'épitope étudié grâce à la réaction immunologique de reconnaissance, voire de neutralisation, de la protéine étrangère par les anticorps correspondants induits par ces séquences chez l'hôte vivant.

7. Procédé selon l'une quelconque des revendications 1 à 6, caractérisé en ce que l'analyse de celles des séquences d'acides aminés qui portent l'épitope requis pour la fabrication du polypeptide vaccinant comprend également la détermination du degré d'hydrophilie desdites séquences, ou toute autre propriété qui indique son accessibilité possible à un anticorps.

8. Procédé selon l'une quelconque des revendications 1 à 7, étendu à l'analyse des séquences peptidiques du "soi immunitaire" qui ne sont pas présentes dans le "soi somatique" du même hôte, le procédé étant appliqué aux molécules du système immunitaire elles-mêmes (particulièrement anticorps et récepteurs des cellules T), de façon à déterminer la séquence des peptides dérivés de ces molécules et susceptibles de jouer un rôle régulateur dans le système immunitaire, pour ensuite synthétiser des peptides ou polypeptides qui pourront être utilisés comme vaccins ou adjuvants de vaccins dans le traitement des infections ou désordres internes de l'organisme de l'hôte commun.

## Claims

1. Procedure for the analysis of peptide sequences carrying an epitope characteristic of a protein foreign to the organism of a host belonging to a defined species, for the subsequent production of immunogenic peptides or polypeptides containing these peptide sequences and possessing properties of vaccines against this foreign protein or against the antigen which it bears, characterized by the following combination of steps:
   - the determination and identification of the family of the oligopeptides having a length superior to 3 amino acids which are contained in the amino acid sequence constituting this foreign protein,
   - the comparison of each of the oligopeptides of this family with each of the oligopeptides of the same length of all of the families of oligopeptides contained in the proteins for which the amino acid sequences are known and which are available in a pre-established catalogue of these proteins, all of these proteins being characterized as the "somatic self" of the corresponding species,
   - the identification of those of the oligopeptides (non coincident oligopeptides) of the family of the foreign protein for with few, and preferably no, coincidences exist with the oligopeptides available in the catalogue,
   - the search, within the foreign protein, for sequences formed of non coincident oligopeptides which mutually overlap, and,
   - the analysis among these sequences of succesive mutually overlapping and non coincident oligopeptides of at least one sequence of amino acids carrying the isotope required for the production of the polypeptide vaccine.

2. Procedure according to claim 1, characterized in that the above-mentioned oligopeptides are tetrapeptides.

3. Procedure according to claim 1 or claim 2, characterized in that the step involving the search for sequences composed of overlapping oligopeptides comprises the determination of those peptides which contain from 6 to 50 (especially from 6 to 30) amino acids.

4. Procedure according to any one of the claims 1 to 3, characterized in that the step of determination and identification of the family of the oligopeptides of the said foreign protein includes the chemical sequencing of this foreign protein, the identification of the said family of oligopeptides then being an immediate consequence of this chemical sequencing.

5. Procedure according to any one of the claims 1 to 3, characterized in that the step involving the determination of the family of the oligopeptides of the said foreign protein comprises the chemical sequencing of the polynucleotide sequence already known to code for the said foreign protein, the identification of the said family of oligopeptides then being an immediate consequence of the chemical sequencing of the polynucleotide sequence by the intermediary of the genetic code.

6. Procedure according to any one of the claims 1 to 5, characterized in that the step of analysis among the successive non coincident and mutually overlapping oligopeptide sequences of one or several of these sequences of amino acids presumed to carry the above-mentioned epitope comprises the chemical synthesis of one or several of these non overlapping oligopeptide sequences, the immunization of an appropriate live host, foreign to the species under study with these oligopeptide sequences and the selection of those of the sequences which contain the epitope under study as deduced from the immunological reaction of recognition, and even of neutralization of the foreign protein by the corresponding antibodies induced by these sequences in the live host.

**7.** Procedure according to any one of the claims 1 to 6, characterized in that the analysis of those of the amino acid sequences which carry the epitope required for the production of the polypeptide vaccine also includes the determination of the degree of hydrophilicity of the said sequences or of any other property which indicates that it may be accessible to the antibody.

**8.** Procedure according to any one of the claims 1 to 7, extended to the analysis of the peptide sequences of the "immune self" which are not present in the "somatic self" of the same host, the procedure being applied to molecules of the immune system itself (particularly antibodies and T cell receptors), so as to determine the sequence of the peptides derived from these molecules which are likely to play a regulatory role in the immune system in order subsequently to synthesize peptides or polypeptides which will be utilizable as vaccines or adjuvants to vaccines in the treatment of the diseases or internal disorders of the organism of the common host.

**Patentansprüche**

**1.** Verfahren zur Analyse auf Peptidsequenzen, die ein Epitop, das von den T-Lymphozyten erkannt werden kann und das für ein fremdes Protein eines Wirtsorganismus, der einer bestimmten Spezies angehört, charakteristisch ist, tragen, zur späteren Herstellung von immunogenen Peptiden oder Polypeptiden, die diese Peptidsequenzen enthalten und Impfeigenschaften gegen dieses fremde Protein oder gegen das es tragende Antigen besitzen, **gekennzeichnet** durch die Kombination der folgenden Schritte:
- Bestimmung und Identifizierung der Oligopeptidfamilie mit einer Länge von mehr als 3 Aminosäuren, die in der dieses fremde Protein bildenden Aminosäuresequenz enthalten sind,
- Vergleich jedes der Oligopeptide dieser Familie mit jedem der Oligopeptide gleicher Länge aus der Gesamtheit der Oligopeptidfamilien, die in den Proteinen enthalten sind, deren Aminosäuresequenzen bekannt sind, und die aus einem vorher zusammengestellten Katalag dieser Proteine verfügbar sind, wobei alle diese Proteine für die "somatische Eigenschaft" der entsprechenden Spezies charakteristisch sind,
- Identifizierung derjenigen Oligopeptide (nicht-übereinstimmende Oligopeptide) der Familie des fremden Proteins, für welche nur geringe Übereinstimmungen und bevorzugt keine übereinstimmung mit den aus diesem Katalag verfügbaren Oligopeptiden bestehen,
- Suche im Kernstück des fremden Proteins nach Sequenzen, die aus nicht-übereinstimmenden Oligopeptiden, die gegenseitig überlappen, gebildet sind, und
- Analyse unter den aus aufeinanderfolgenden, nicht-übereinstimmenden und gegenseitig überlappenden Oligopeptiden gebildeten Sequenzen auf mindestens eine Aminosäuresequenz, die das zur Herstellung des impfaktiven Polypeptids benötigte Epitop trägt.

**2.** Verfahren nach Anspruch 1, dadurch gekennzeichnet, daß die genannten Oligopeptide Tetrapeptide sind.

**3.** Verfahren nach Anspruch 1 oder 2, dadurch gekennzeichnet, daß man bei der Suche nach Sequenzen, die aus überlappenden Oligopeptiden gebildet sind, diejenigen unter ihnen, die mindestens 6 bis 50 (insbesondere 6 bis 30) Aminosäuren umfassen, bestimmt.

**4.** Verfahren nach einem der Ansprüche 1 bis 3, dadurch gekennzeichnet, daß man bei der Bestimmung und Identifizierung der Oligopeptidfamilie des fremden Proteins, chemisch das fremde Protein sequenziert, wobei die Identifizierung der Oligopeptidfamilie dann unmittelbar ausgehend von der chemischen Sequenzierung vorgenommen wird.

**5.** Verfahren nach einem der Ansprüche 1 bis 3, dadurch gekennzeichnet, daß man bei der Bestimmung der Oligopeptidfamilie des fremden Proteins chemisch die Polynucleotidsequenz, von der zuvor bekannt ist, daß sie für das fremde Protein codiert, sequenziert, wobei man die Identifizierung durch Vermittlung des genetischen Codes unmittelbar von der chemischen Sequenzierung der Polynucleotidsequenz vornimmt.

**6.** Verfahren nach einem der Ansprüche 1 bis 5, dadurch gekennzeichnet, daß man bei der Analyse unter den aus aufeinanderfolgenden, gegenseitig überlappenden und nicht-übereinstimmenden Oligopeptiden gebildeten Sequenzen auf eine oder mehrere dieser Aminosäuresequenzen, die vermutlich das genannte Epitop tragen, chemisch eine oder mehrere der aus nicht-überlappenden Oligopeptiden gebildeten Sequenzen synthetisiert, einen geeigneten lebenden, der untersuchten Spezies fremden Wirt mit diesen Oligopeptidsequenzen immunisiert, und diejenigen Sequenzen, die das untersuchte Epitop tragen, dank einer immunologischen Erkennungsreaktion, nämlich durch Neutralisation des fremden Proteins durch entsprechende Antikörper, die durch diese Sequenzen bei dem lebenden Wirt hervorgerufen wurden, auswählt.

7.  Verfahren nach einem der Ansprüche 1 bis 6, dadurch gekennzeichnet, daß man bei der Analyse auf Aminosäuresequenzen, die das zur Herstellung des impfaktiven Polypeptids benötigte Epitop tragen, ebenso den Hydrophiliegrad der Sequenzen oder jede andere Eigenschaft, die dessen mögliche Erreichbarkeit für einen Antikörper anzeigt, bestimmt.

8.  Verfahren nach einem der Ansprüche 1 bis 7, dadurch gekennzeichnet, daß man weiterhin die Peptidsequenzen der "immunologischen Eigenschaft", die in der "somatischen Eigenschaft" des gleichen Wirts nicht vorhanden sind, analysiert, wobei das Verfahren auf die Moleküle des Immunsystems selbst (insbesondere Antikörper und Rezeptoren der T-Zellen) so angewendet wird, daß man die Sequenz der Peptide, die sich von diesen Molekülen ableiten und eine Rolle als Regulator im Immunsystem spielen können, bestimmt, um dann Peptide oder Polypeptide zu synthetisieren, die als Impfstoffe oder Hilfs-Impfstoffe bei der Behandlung von Infektionen oder inneren Störungen des gemeinsamen Wirtsorganismus verwendet werden können.

FIG.1

FIG.2

FIG.3

FIG. 4

FIG.5